# FASCICULE DE BREVET EUROPEEN

(11) **EP 0 740 933 B1**
(45) Date de publication et mention de la délivrance du brevet: **26.03.1997**
(21) Numéro de dépôt: 96400772.8
(22) Date de dépôt: 10.04.1996
(51) Int. Cl.: A61K 7/48, A61K 7/043

(54) **Composition de vernis à ongles comprenant un polyester réticulé**
Nagellackzusammensetzung, die einen vernetzten Polyester enthält
Nail varnish composition containing a crosslinked polyester

(30) Priorité: 05.05.1995 FR 9505421
(43) Date de publication de la demande: 06.11.1996
(73) Titulaire: L'OREAL, 75008 Paris (FR)
(72) Inventeur: Junino, Alex, 93190 Livry Gargan (FR); Ramin, Roland, 91760 Itteville (FR)
(74) Mandataire: Dodin, Catherine

(56) Documents cités:
- AU-D- 6 445 865
- US-A- 3 925 295

## Description

La présente invention a pour objet une composition de vernis à ongles, incolore ou colorée, comprenant outre les constituants habituels, un polyester réticulé comme plastifiant.

Parmi les principales caractéristiques que doivent posséder les vernis à ongles, on doit tout particulièrement citer l'absence d'irritation de la peau et des ongles, une bonne application, l'obtention d'un film homogène d'excellente brillance, un temps de séchage du film rapide, mais aussi une bonne adhérence sur la surface de l'ongle, une certaine flexibilité et une bonne résistance du film en vue d'éviter les craquelures et son écaillement. Ces caractéristiques sont généralement dénommées: caractéristiques cosmétiques.
De façon générale, on utilise à l'heure actuelle, pour conférer une bonne adhérence et une bonne flexibilité du film, des matières filmogènes telles que la nitrocellulose associée, si nécessaire, à un autre polymère tel qu'une résine acrylique ou une résine alkyde et à des plastifiants (voir à ce sujet notamment le document FR-A-2679445).
Les plastifiants couramment utilisés sont les plastifiants de type phtalate comme le phtalate de dibutyle, de type citrate comme l'acétyl citrate de tributyle, de type ester de glycol comme l'ester de néopentylglycol ou de propylène glycol, de type benzoate de glycéryle et enfin le camphre.
Ces agents plastifiants permettent de régler la flexibilité du film sans affaiblir sa résistance physique.
De nos jours, on souhaite toutefois utiliser dans les vernis d'autres plastifiants que les phtalates, qui peuvent être impliqués dans les allergies mais aussi que le camphre qui, par sa volatilité, ne permet pas d'avoir des caractéristiques de vernis constantes, tout en conservant des caractéristiques cosmétiques adéquates.

De façon surprenante, la Demanderesse a trouvé qu'on pouvait utiliser un polyester réticulé comme plastifiant dans les compositions de vernis à ongles à milieu organique, sans modifier les propriétés cosmétiques du vernis et tout en obtenant une composition stable, ainsi qu'un film souple et brillant.

De façon plus précise, l'invention se rapporte à une composition cosmétique, notamment de vernis à ongles, comprenant au moins une matière filmogène, au moins un plastifiant et un milieu solvant, caractérisée en ce que le plastifiant comprend au moins un polyester réticulé issu de la polycondensation d'acide adipique, de diéthylène glycol et d'un polyol ayant au moins trois groupements hydroxyle.

De préférence, le polyol du polyester réticulé comporte trois groupements hydroxyle, comme le glycérol.

On peut en particulier citer comme polyester réticulé d'acide adipique, de diéthylène glycol et de glycérol celui commercialisé sous la référence "LEXOREZ 100" par la société INOLEX.

Le polyester réticulé peut être présent dans la composition selon l'invention à une teneur allant de 0,1 % à 15 % en poids par rapport au poids total de la composition.

Le polyester réticulé peut être utilisé seul ou éventuellement associé à un autre plastifiant connu de l'homme de l'art.
Les plastifiants utilisables en association avec le polyester réticulé peuvent être choisis, par exemple, parmi ceux cités dans le document FR-A-2679445 tels que les phtalates de dibutyle, de dioctyle, de di-isobutyle, de diméthoxyéthyle ; les benzoates de benzyle, de glycéryle ; les citrates de triéthyle, de tributyle, l'acétyl-citrate de tributyle ; les phosphates de tributyle, de triphényle ; les glycols ; le camphre ainsi que leurs dérivés et leurs mélanges. Ces plastifiants peuvent représenter au plus 50 % en poids de la quantité totale de plastifiant.

Les matières filmogènes selon l'invention comprennent toute matière filmogène connue de l'homme du métier, et en particulier la nitrocellulose éventuellement associée à une résine alkyde, une résine polyester, une résine acrylique, une résine polyuréthanne, une résine polyamide, une résine vinylique, et de façon générale à toute résine compatible avec le milieu. Ces résines permettent d'augmenter le pouvoir filmogène de la nitrocellulose et améliorent le brillant ainsi que l'adhérence des films.
Il est en outre possible de remplacer, totalement ou partiellement la nitrocellulose par une résine polyvinylique telle que le butyrale de polyvinyle ou par l'acétobutyrate de cellulose.

On a de plus constaté que lorsque la composition selon l'invention comprend comme seul agent plastifiant un polyester réticulé selon l'invention, et comme matière filmogène de la nitrocellulose, on obtient après application sur l'ongle un film pelliculable

Selon l'invention, le milieu solvant peut être essentiellement constitué par un mélange de divers solvants organiques volatils, ceci en vue d'obtenir des temps de séchage relativement courts.
Les solvants utilisés sont de préférence compatibles avec les résines utilisées afin d'assurer leur dissolution. Parmi ces solvants, qui sont ceux classiquement utilisés dans les vernis à ongles, on peut citer les cétones, comme l'acétone, la méthyl-éthylcétone, la méthyl-isobutyl-cétone ; les éthers de glycol ; les alcools comme l'éthanol, le n-butanol, le n-propanol, l'isopropanol ; les acétates comme l'acétate de butyle, d'éthyle, d'isopropyle, l'acétate de méthoxy-2-éthyle ; les hydrocarbures linéaires ou ramifiés tels que l'hexane ou l'octane ; ou encore les hydrocarbures aromatiques tels que le xylène et le toluène.

Dans un mode préféré de réalisation, la composition selon l'invention comprend en particulier, en poids, de 0,1 à 15 % de plastifiant, de 5 à 35 % de matière filmogène et de 50 à 94,9 % de milieu solvant, par rapport au poids total de la composition.

Lorsque le vernis à ongles selon l'invention est un vernis coloré, on peut y incorporer au moins un pigment de nature organique ou inorganique, un colorant, une laque, et/ou un agent nacrant bien connu de l'homme du métier.
Par ailleurs, en vue d'éviter la sédimentation des pigments, certains agents rhéologiques thixotropes peuvent être employés, tels que les argiles du type bentonite, hectorite ou montmorillonite. Ces agents servent en outre d'épaississants.
Les compositions selon l'invention peuvent par ailleurs contenir des adjuvants couramment utilisés dans les vernis à ongles. Parmi ces adjuvants, on peut citer les filtres U.V. tels que les dérivés de benzophénone et le 2-cyano-3, 3-diphényl acrylate d'éthyle, des silicones et des agents fluorés.
Bien entendu l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

L'invention a également pour objet l'utilisation d'un polyester réticulé tel que défini précédemment comme agent plastifiant dans une composition cosmétique, notamment de vernis à ongles, comprenant au moins une matière filmogène et au moins un milieu solvant.

On va maintenant donner à titre d'illustration et sans aucun caractère limitatif plusieurs exemples de composition de vernis à ongles selon l'invention.

### Exemple 1

On a préparé un vernis à ongles non coloré ayant la composition suivante :

| | |
|---|---|
| - Nitrocellulose | 20 g |
| - Polyester réticulé (Lexorez 100 de la société INOLEX) | 8 g |
| - Bentonite | 1 g |
| - Additifs | 0,5 g |
| - Solvant (acétate d'éthyle, acétate de butyle) | qsp 100 g |

Le vernis s'étale facilement et donne, après séchage, un film souple et brillant.

### Exemple 2

On a préparé un vernis à ongles non coloré ayant la composition suivante :

| | |
|---|---|
| - Nitrocellulose | 15 g |
| - Polyester réticulé (Lexorez 100 de la société INOLEX) | 6 g |
| - Bentone | 1 g |
| - Additifs | 3,5 g |
| - Résine tosylamide/formaldéhyde | 12 g |
| - Solvant (acétate d'éthyle, acétate de butyle) | qsp 100 g |

Le vernis s'étale facilement et donne, après séchage, un film souple et brillant.

### Exemple 3

On a préparé un vernis à ongles non coloré ayant la composition suivante :

| | |
|---|---|
| - Nitrocellulose | 15 g |
| - Polyester réticulé (Lexorez 100 de la société INOLEX) | 8 g |
| - Bentone | 1 g |
| - Additifs | 1 g |
| - Acétyl-citrate de tributyle | 6 g |
| - Solvants (acétate d'éthyle, acétate de butyle) | qsp 100 g |

Le vernis s'étale facilement et donne, après séchage, un film souple et brillant.

## Revendications

1. Composition cosmétique comprenant au moins une matière filmogène, au moins un plastifiant et un milieu solvant, caractérisée en ce que le plastifiant comprend au moins un polyester réticulé issu de la polycondensation d'acide adipique, de diéthylène glycol et d'un polyol ayant au moins trois groupements hydroxyle.

2. Composition selon la revendication 1, caractérisée par le fait que le polyol du polyester comporte trois groupements hydroxyle.

3. Composition selon l'une des revendications précédentes, caractérisée par le fait que le polyol est le glycérol.

4. Composition selon l'une des revendications précédentes, caractérisée par le fait que le polyester réticulé est présent dans la composition à une teneur allant de 0,1 % à 15 % en poids par rapport au poids total de la composition.

5. Composition selon l'une des revendications précédentes, caractérisée par le fait que le polyester réticulé est utilisé associé à un autre plastifiant connu de l'homme de l'art.

6. Composition selon la revendication 5, dans laquelle l'autre plastifiant est choisi parmi les phtalates de dibutyle, de dioctyle, de di-isobutyle, de diméthoxyéthyle ; les benzoates de benzyle, de glycéryle ; les citrates de triéthyle, de tributyle, l'acétyl-citrate de tributyle ; les phosphates de tributyle, de triphényle ; les glycols ; le camphre ainsi que leurs dérivés et leurs mélanges.

7. Composition selon l'une des revendications précédentes, caractérisée par le fait que la matière filmogène est choisie parmi la nitrocellulose, le butyrale de polyvinyle et l'acétobutyrate de cellulose.

8. Composition selon l'une des revendications précédentes, caractérisée par le fait que la matière filmogène comprend en outre une résine choisie parmi une résine alkyde, une résine polyester, une résine acrylique, une résine polyuréthanne, une résine polyamide, une résine vinylique, et de façon générale toute résine compatible avec le milieu.

9. Composition selon l'une des revendications précédentes, caractérisée par le fait qu'elle comprend, en poids, de 0,1 à 15 % de plastifiant, de 5 à 35 % de matière filmogène et de 50 à 94,9 % de milieu solvant, par rapport au poids total de la composition.

10. Composition selon l'une des revendications précédentes, se présentant sous la forme d'un vernis à ongles.

11. Utilisation d'un polyester réticulé issu de la polycondensation d'acide adipique, de diéthylène glycol et d'un polyol ayant au moins trois groupements hydroxyle, comme agent plastifiant dans une composition cosmétique comprenant au moins une matière filmogène et au moins un milieu solvant.

12. Utilisation selon la revendication 11, caractérisée par le fait que le polyol du polyester comporte trois groupements hydroxyle.

13. Utilisation selon l'une des revendications 11 à 12, caractérisée par le fait que le polyol est le glycérol.

14. Utilisation selon l'une des revendications 11 à 13, dans une composition de vernis à ongles.

## Claims

1. Cosmetic composition comprising at least one film-forming material, at least one plasticizer and a solvent medium, characterized in that the plasticizer comprises at least one crosslinked polyester derived from the polycondensation of adipic acid, diethylene glycol and a polyol having at least three hydroxyl groups.

2. Composition according to Claim 1, characterized in that the polyol of the polyester contains three hydroxyl groups.

3. Composition according to either of the preceding claims, characterized in that the polyol is glycerol.

4. Composition according to one of the preceding claims, characterized in that the crosslinked polyester is present in the composition in a content ranging from 0.1% to 15% by weight relative to the total weight of the composition.

5. Composition according to one of the preceding claims, characterized in that the crosslinked polyester is used combined with another plasticizer known to those skilled in the art.

6. Composition according to Claim 5, in which the other plasticizer is chosen from dibutyl, dioctyl, diisobutyl and dimethoxyethyl phthalates; benzyl and glyceryl benzoates; triethyl and tributyl citrates, tributyl acetyl citrate; tributyl and triphenyl phosphates; glycols and camphor, as well as derivatives and mixtures thereof.

7. Composition according to one of the preceding claims, characterized in that the film-forming material is chosen from nitrocellulose, polyvinyl butyral and cellulose acetobutyrate.

8. Composition according to one of the preceding claims, characterized in that the film-forming material also comprises a resin chosen from an alkyd resin, a polyester resin, an acrylic resin, a polyurethane resin, a polyamide resin, a vinyl resin and, in general, any resin compatible with the medium.

9. Composition according to one of the preceding claims, characterized in that it comprises by weight, from 0.1 to 15% of plasticizer, from 5 to 35% of film-forming material and from 50 to 94.9% of solvent medium, relative to the total weight of the composition.

10. Composition according to one of the preceding claims, which is in the form of a nail varnish.

11. Use of a crosslinked polyester derived from the polycondensation of adipic acid, diethylene glycol and a polyol having at least three hydroxyl groups, as plasticizer in a cosmetic composition comprising at least one film-forming material and at least one solvent medium.

12. Use according to Claim 11, characterized in that the polyol of the polyester contains three hydroxyl groups.

13. Use according to either of Claims 11 and 12, characterized in that the polyol is glycerol.

14. Use according to one of Claims 11 to 13, in a nail varnish composition.

## Patentansprüche

1. Kosmetische Zusammensetzung, die mindestens ein filmbildendes Material, mindestens einen Weichmacher und mindestens ein Lösungsmittelmedium enthält,
**dadurch gekennzeichnet, daß**
der Weichmacher mindestens einen vernetzten Polyester umfaßt, der aus der Polykondensation von Adipinsäure, Diethylenglykol und einem Polyol mit mindestens drei Hydroxygruppen stammt.

2. Zusammensetzung nach Anspruch 1, dadurch gekennzeichnet, daß das Polyol des Polyesters drei Hydroxygruppen enthält.

3. Zusammensetzung nach einem der vorhergehenden Ansprüche, dadurch gekennzeichnet, daß das Polyol Glycerin ist.

4. Zusammensetzung nach einem der vorhergehenden Ansprüche, dadurch gekennzeichnet, daß der vernetzte Polyester in der Zusammensetzung in einem Mengenanteil von 0,1 bis 15 Gew.-%, bezogen auf das Gesamtgewicht der Zusammensetzung, vorliegt.

5. Zusammensetzung nach einem der vorhergehenden Ansprüche, dadurch gekennzeichnet, daß der vernetzte Polyester in Kombination mit einem weiteren dem Fachmann bekannten Weichmacher verwendet wird.

6. Zusammensetzung nach Anspruch 5, worin der weitere Weichmacher ausgewählt ist unter: Dibutylphthalat, Dioctylphthalat, Diisobutylphthalat und Dimethoxyethylphthalat, Benzylbenzoat und Glycerylbenzoat, Triethylcitrat, Tributylcitrat und Acetyltributylcitrat, Tributylphosphat und Triphenylphosphat, Glykolen, Campher sowie deren Derivaten und deren Gemischen.

7. Zusammensetzung nach einem der vorhergehenden Ansprüche, dadurch gekennzeichnet, daß das filmbildende Material unter Nitrocellulose, Polyvinylbutyral und Celluloseacetobutyrat ausgewählt ist.

8. Zusammensetzung nach einem der vorhergehenden Ansprüche, dadurch gekennzeichnet, daß das filmbildende Material ferner ein Harz enthält, das unter einem Alkydharz, Polyesterharz, Acrylharz, Polyurethanharz, Polyamidharz, Vinylharz und allgemein einem beliebigen mit dem Medium kompatiblen Harz ausgewählt ist.

9. Zusammensetzung nach einem der vorhergehenden Ansprüche, dadurch gekennzeichnet, daß sie 0,1 bis 15 Gew.-% Weichmacher, 5 bis 35 Gew.-% filmbildendes Material und 50 bis 94,9 Gew.-% Lösungsmittelmedium, bezogen auf das Gesamtgewicht der Zusammensetzung, enthält.

10. Zusammensetzung nach einem der vorhergehenden Ansprüche, die in Form eines Nagellacks vorliegt.

11. Verwendung eines vernetzten Polyesters, der aus der Polykondensation von Adipinsäure, Diethylenglykol und einem Polyol mit mindestens drei Hydroxygruppen stammt, als Weichmacher in einer kosmetischen Zusammensetzung, die mindestens ein filmbildendes Material und mindestens ein Lösungsmittelmedium enthält.

12. Verwendung nach Anspruch 11, dadurch gekennzeichnet, daß das Polyol des Polyesters drei Hydroxygruppen enthält.

13. Verwendung nach einem der Ansprüche 11 oder 12, dadurch gekennzeichnet, daß das Polyol Glycerin ist.

14. Verwendung nach einem der Ansprüche 11 bis 13 in einer Nagellackzusammensetzung.
